# EUROPÄISCHE PATENTANMELDUNG

(11) **EP 2 525 199 A1**
(43) Veröffentlichungstag der Anmeldung: **21.11.2012**
(21) Anmeldenummer: 11166639.2
(22) Anmeldetag: 19.05.2011
(51) Int. Cl.: G01K 7/02, G01K 7/04, G01K 13/00, A61C 1/08, A61C 1/14

(54) **Medizinisches, insbesondere dentales, Diagnose- oder Behandlungselement**

(71) Anmelder: W & H Dentalwerk Bürmoos GmbH, 5111 Bürmoos (AT)
(72) Erfinder: Pruckner, Christian, 1190, Wien (AT)
(74) Vertreter: Benda, Ralf

(57) **Zusammenfassung**

Es wird ein medizinisches, insbesondere dentales, Diagnose- oder Behandlungselement (1, 4) offenbart, welches umfasst: Eine Vorrichtung (60, 4A) zum direkten oder indirekten Wirken auf ein Zielgebiet und eine Daten- und / oder Energietransfereinheit (41) zur berührungslosen Daten- und / oder Energieübertragung, wobei die Daten- und / oder Energietransfereinheit (41) zumindest eine erste Spule (5 ― 5C, 6, 6'), Wicklung oder Antenne aufweist, welche zur Daten- und / oder Energieübertragung berührungslos, insbesondere induktiv, mit einer zweiten Spule (5 ― 5C, 6, 6'), Wicklung oder Antenne koppelbar ist, wobei die erste Spule (5 ― 5C, 6, 6'), Wicklung oder Antenne ein erstes Material (M1) aufweist, welches Teil einer Thermoelementverbindung (21) zur Messung der Temperatur im Diagnose- oder Behandlungselement (1, 4) oder eines Bauteils des Diagnose- oder Behandlungselements (1, 4) ist. Des Weiteren wird ein entsprechendes Verfahren zur Messung der Temperatur mittels eines medizinischen, insbesondere dentalen, Diagnose- oder Behandlungselements (1, 4) beschrieben.

## Beschreibung

Die vorliegende Erfindung betrifft ein medizinisches, insbesondere dentales, Diagnose- oder Behandlungselement mit einer Vorrichtung zum direkten oder indirekten Wirken auf ein Zielgebiet und einer Sende- und / oder Empfangseinheit zur berührungslosen Daten- und / oder Energieübertragung.

Ein derartiges Diagnose- oder Behandlungselement ist zum Beispiel aus der Patentanmeldung EP 22 33 103 A2 bekannt. Das Diagnose- oder Behandlungselement in Form eines Handgriffelements umfasst eine Außenhülse, eine in der Außenhülse angeordnete und in eine Antriebsbewegung versetzbare Hohlwelle zur Aufnahme eines Behandlungswerkzeugs und eine erste Spule zur induktiven Kopplung mit einer eine zweite Spule umfassenden Speichereinheit des Behandlungswerkzeugs. Die beiden Spulen sind Teil einer Sende- und / oder Empfangseinheit zur berührungs- oder drahtlosen, induktiven Daten- und / oder Energieübertragung.

Der vorliegenden Erfindung liegt die Aufgabe zugrunde ein medizinisches, insbesondere dentales, Diagnose- oder Behandlungselement, wie es aus der oben genannten Patentanmeldung bekannt ist, derart weiter zu entwickeln, dass es mit einer zusätzlichen, bis dato nicht durchführbaren Funktion ausgestattet ist, wobei insbesondere die im Diagnose- oder Behandlungselement vorhandenen Bauteile für die zusätzliche Funktion genützt werden sollen, so dass trotz der äußerst beengten Platzverhältnisse im Inneren eines Diagnose- oder Behandlungselements, insbesondere in dessen Kopfbereich, die zusätzliche Funktion in das Diagnose- oder Behandlungselement implementierbar ist.

Diese Aufgabe wird gemäß der vorliegenden Erfindung durch ein medizinisches, insbesondere dentales, Diagnose- oder Behandlungselement mit den Merkmalen des Anspruchs 1 gelöst. Das Diagnose- oder Behandlungselement weist dem gemäß auf: Eine Vorrichtung zum direkten oder indirekten Wirken auf ein Zielgebiet und eine Daten- und / oder Energietransfereinheit zur berührungslosen Daten- und / oder Energieübertragung, wobei die Daten- und / oder Energietransfereinheit zumindest eine erste Spule, Wicklung oder Antenne aufweist, welche zur Daten- und / oder Energieübertragung berührungslos, insbesondere induktiv, mit einer zweiten Spule, Wicklung oder Antenne koppelbar ist, und wobei die erste Spule, Wicklung oder Antenne ein erstes Material aufweist, welches Teil einer Thermoelementverbindung zur Messung eines Temperaturmesswerts des Diagnose- oder Behandlungselements oder eines Bauteils des Diagnose- oder Behandlungselements ist.

Der Anspruch 13 bezieht sich auf ein entsprechendes Verfahren zur Messung eines Temperaturmesswerts mittels eines medizinischen, insbesondere dentalen, Diagnose- oder Behandlungselements.

Das Diagnose- oder Behandlungselement nutzt somit die aus dem Stand der Technik bekannte Sende- und / oder Empfangseinheit zur berührungslosen, induktiven Daten- und / oder Energieübertragung, insbesondere deren Spule, Wicklung oder Antenne, zur Implementierung einer zusätzlichen Funktion in dem Diagnose- oder Behandlungselement, nämlich zum Messen der Temperatur im Diagnose- oder Behandlungselement. Dazu wird die Spule, Wicklung oder Antenne als Thermoelement oder zumindest als Teil eines Thermoelements ausgebildet. In vorteilhafter Weise wird damit für die Temperaturmessung im Diagnose- oder Behandlungselement kein eigener Temperatursensor benötigt, sondern es werden bereits im Diagnose- oder Behandlungselement vorhandenen Bauteile für die Temperaturmessung genützt.

Der Begriff Thermoelement umfasst eine Vorrichtung, die durch Thermoelektrizität Wärme in elektrische Energie wandelt. Insbesondere weist ein Thermoelement zwei unterschiedliche und an einem Ende miteinander verbundene, elektrisch leitende Materialien, insbesondere Metalle oder Metalldrähte auf, um damit Temperaturmessungen durchführen zu können. Ein Thermoelement weist somit bevorzugt einen bimetallischen Thermokontakt auf. Eine Temperaturdifferenz an den Enden der Metalle oder Metalldrähte erzeugt aufgrund des Seebeck-Effekts innerhalb der beiden Metalle eine elektrische Ladungstrennung oder eine elektrische Thermospannung. Diese elektrische Potentialdifferenz ist annähernd proportional zur Temperaturdifferenz an den Enden der Metalle. Um die absolute Temperatur an den Metallen oder Drahtenden bestimmen zu können, müssen weitere Maßnahmen ergriffen werden, zum Beispiel die Messung der Umgebungstemperatur, zu der die Temperaturdifferenz an den Enden der Metalle addiert wird. Da der Aufbau und die Funktion eines Thermoelements bekannt sind, wird darauf nicht weiter eingegangen.

Der Begriff Diagnose- oder Behandlungselement umfasst insbesondere mit einer Hand greifbare medizinische oder dentale Geräte, zum Beispiel gerade oder pistolenförmige Diagnose- oder Behandlungselemente oder Handgriffe, gebogene Diagnose- oder Behandlungselemente oder Handgriffe, die im Dentalbereich oftmals als Winkelstücke bezeichnet werden, Teile von Diagnose- oder Behandlungselementen, insbesondere einen Kopfabschnitt eines Diagnose- oder Behandlungselements, der zum Beispiel mit davon lösbaren Griffabschnitten verbindbar ist, sowie Kupplungen, Adapter, Verbindungsstücke und Antriebseinheiten, zum Beispiel elektrische oder pneumatische Motoren. Unter der Bezeichnung Diagnose- oder Behandlungselement werden des Weiteren sowohl schnurlose Diagnose- oder Behandlungselemente verstanden, insbesondere mit einer austauschbaren oder aufladbaren Energiequelle, als auch Diagnose- oder Behandlungselemente, die eine Versorgungsleitung und eine damit verbundene Steuer-, Regel- und / oder Versorgungseinheit umfassen.

Unter dem Begriff ,Vorrichtung zum direkten oder indirekten Wirken auf ein Zielgebiet' wird insbesondere jede Vorrichtung verstanden, die unter Nutzung von Energie und / oder Abgabe von Materie und / oder Energie auf eine Zielgebiet, insbesondere ein tierisches oder menschliches Gewebe oder ein tierisches oder menschliches Gewebe substituierendes Material, zum Beispiel eine Prothese, einwirkt, insbesondere therapeutisch oder diagnostisch einwirkt. Das Einwirken der Vorrichtung auf ein Zielgebiet erfolgt zum Beispiel durch das Beaufschlagen eines Zielgebiets mit Energie und / oder Materie, durch Be- oder Durchleuchten, Scannen, Abtragen von Gewebe oder Gewebeersatzmaterial, insbesondere von Hartgewebe und Knochen, das Entfernen von Gewebe oder Gewebeersatzmaterial, zum Beispiel durch Wegblasen, Wegwaschen, Ätzen, Auflösen, etc., durch das Aufbringen eines Ersatzmaterials, Füllmaterials, durch die Abgabe eines Wirkstoffs, Medikaments oder Anästhetikums, durch das Auslösen einer chemischen oder physikalischen Reaktion in oder an einem Gewebe oder Gewebeersatzmaterial, usw. Die Vorrichtung zum Wirken auf ein Zielgebiet umfasst dem gemäß direkt auf das Zielgebiet wirkende Vorrichtungen, zum Beispiel Werkzeuge, insbesondere abrasive Werkzeuge wie Bohrer, Fräsen, Sägen, Feilen, Reamer, Skalpelle, etc. oder Elektroden als auch indirekt auf das Zielgebiet wirkende Vorrichtungen, zum Beispiel Antriebe für direkt auf das Zielgebiet wirkende Vorrichtungen, zum Beispiel elektrische oder pneumatische Antriebe oder Teile davon, insbesondere Wellen, Getriebe, Laufräder, etc., Düsen oder Abgabeöffnungen zur Abgabe von Materie auf das Zielgebiet, Strahlungsquellen, optische Elemente für Strahlung, insbesondere Laserstrahlung, Lichtleiter, usw.

Gemäß einem bevorzugten Ausführungsbeispiel umfasst das erste Material der Spule, Wicklung oder Antenne, das Teil des Thermoelements oder der Thermoelementverbindung ist, ein elektrisch leitendes Material, insbesondere ein Metall, zum Beispiel Kupfer, oder einen Metalldraht, zum Beispiel einen Kupferdraht, insbesondere einen Kupferdraht wie er herkömmlicherweise als elektrischer Leiter oder als Leiterbahn auf Platinen verwendet ist. Das andere elektrisch leitende Material des Thermoelements oder der Thermoelementverbindung umfasst zum Beispiel eine Kupfer-Nickel-Legierung (Konstantan) oder andere Legierungen, welche zum Beispiel Nickel, Chrom, Eisen oder Kupfer enthalten.

Gemäß einem besonders Platz sparendem Ausführungsbeispiel weist die erste Spule, Wicklung oder Antenne ein zweites Material auf, so dass die Thermoelementverbindung durch die Spule, Wicklung oder Antenne gebildet ist.

Gemäß einem alternativen, in der Herstellung sehr einfachem Ausführungsbeispiel weist ein mit der ersten Spule, Wicklung oder Antenne verbundener elektrischer Leiter ein zweites Material auf, so dass die Thermoelementverbindung durch die Spule, Wicklung oder Antenne und den damit verbundenen elektrischen Leiter gebildet ist. Bevorzugt besteht die Spule, Wicklung oder Antenne dabei aus Kupferdraht, mit dem der aus dem zweiten Material bestehende elektrische Leiter verbunden ist.

Gemäß einem bevorzugten Ausführungsbeispiel ist zumindest ein gemeinsames Verarbeitungs- und / oder Auswerteelement für den von der Thermoelementverbindung ermittelten Temperaturmesswert und die Daten und / oder Energie der berührungslosen Daten- und / oder Energieübertragung vorgesehen, insbesondere ein gemeinsamer Filter, Signalteiler, Verstärker oder eine gemeinsame Auswerteschaltung für die Daten. Damit wird in vorteilhafter Weise zumindest ein weiteres im Diagnose- oder Behandlungselement vorhandenes Bauteil für die Temperaturmessung verwendet. Um ein derartiges gemeinsames Verarbeitungs- und / oder Auswerteelement störungsfrei und zuverlässig für den Temperaturmesswert und die Daten und / oder Energie der berührungslosen Daten- und / oder Energieübertragung nützen zu können, ist besonders bevorzugt eine Schaltvorrichtung zur wahlweisen Übertragung und / oder Verarbeitung des von der Thermoelementverbindung ermittelten Temperaturmesswerts und der Daten und / oder Energie der berührungslosen Daten- und / oder Energieübertragung vorgesehen. Die Schaltvorrichtung bewirkt zum Beispiel, dass ein gemeinsames Verarbeitungs- und / oder Auswerteelement zeitlich sequentiell entweder mit dem Temperaturmesswert oder mit den Daten und / oder der Energie der berührungslosen Daten- und / oder Energieübertragung versorgt wird. Die Schaltvorrichtung kann zum Beispiel zum Beispiel als Signalweiche oder als mechanischer oder digitaler Schalter ausgebildet sein.

Vorzugsweise weist das Diagnose- oder Behandlungselement einen Kopfabschnitt auf, von dem die Vorrichtung zum direkten oder indirekten Wirken auf ein Zielgebiet Materie und / oder Energie auf das Zielgebiet abgibt, wobei die Thermoelementverbindung derart angeordnet ist, dass sie die Temperatur zumindest eines Teils des Kopfabschnitts oder eines Bauteils des Kopfabschnitts misst, wobei die Thermoelementverbindung insbesondere in oder an dem Kopfabschnitt angeordnet ist. Da der Kopfabschnitt bei Betrieb des Diagnose- oder Behandlungselements besonders nahe an dem Patienten bzw. an der Präparationsstelle oder dem Zielgebiet angeordnet ist, ist es von Vorteil während des Betriebs die Temperatur zumindest eines Teils des Kopfabschnitts oder eines Bauteils des Kopfabschnitts durch die Thermoelementverbindung bestimmen zu lassen, um Verletzungen des Patienten, zum Beispiel Verbrennungen, zu verhindern.

Gemäß einem bevorzugten Ausführungsbeispiel weist das Diagnose- oder Behandlungselement eine Werkzeughalte- und Lösevorrichtung zur lösbaren Verbindung eines Werkzeugs mit dem Diagnose- oder Behandlungselement auf, wobei die Thermoelementverbindung derart angeordnet ist, dass sie die Temperatur zumindest eines Teils der Werkzeughalte- und Lösevorrichtung oder im Bereich der Werkzeughalte- und Lösevorrichtung misst. Ähnlich wie im vorstehenden Ausführungsbeispiel befinden sich zumindest Teile der Werkzeughalte- und Lösevorrichtung während des Betriebs des Diagnose- oder Behandlungselements sehr nahe an dem Patienten bzw. an der Präparationsstelle oder dem Zielgebiet, so dass eine Temperaturmessung an der Werkzeughalte- und Lösevorrichtung wiederum vorteilhafterweise beiträgt, Verletzungen des Patienten oder auch des Anwenders beim Betätigen der Werkzeughalte- und Lösevorrichtung zu vermeiden.

Gemäß einem weiteren bevorzugten Ausführungsbeispiel weist das Diagnose- oder Behandlungselement eine Steuer- und / oder Regelvorrichtung auf, welche ausgebildet ist, den mittels der Thermoelementverbindung ermittelten Temperaturmesswert mit einem Temperaturgrenzwert zu vergleichen und bei Erreichen oder Überschreiten des Temperaturgrenzwerts das Einwirken der Wirkvorrichtung auf das Zielgebiet zu unterbrechen, insbesondere einen Antrieb für die Wirkvorrichtung anzuhalten, und / oder das Erreichen oder Überschreiten des Temperaturgrenzwerts mittels einer Anzeigevorrichtung anzuzeigen. Beide Maßnahmen verringern in vorteilhafter Weise negative Auswirkungen einer zu starken Erhitzung des Diagnose- oder Behandlungselements oder Teile davon noch zusätzlich.

Gemäß einem anderen Ausführungsbeispiel ist die Sende- und / oder Empfangseinheit insbesondere zur berührungslosen Datenübertragung, gegebenenfalls auch zur berührungslosen Energieübertragung, im Hochfrequenz- oder Radiofrequenzbereich ausgebildet. Der Hochfrequenzbereich umfasst dabei bevorzugt auch den VHF- (very high frequenzy) und den UHF- (ultra high frequenzy) Bereich. Bevorzugt umfasst die Sende- und / oder Empfangseinheit ein RFID-Etikett oder einen RFID-Transponder, welche insbesondere einen elektronischen Chip aufweisen. Besonders bevorzugt umfasst das Diagnose- oder Behandlungselement oder das RFID-Etikett oder der RFID-Transponder oder der elektronische Chip ein mit der Sende- und / oder Empfangseinheit zur berührungslosen Daten- und / oder Energieübertragung, insbesondere mit der ersten Spule, Wicklung oder Antenne, verbundenes, insbesondere mehrfach beschreibbares, Speicherelement, das ausgebildet ist, mittels der Sende- und / oder Empfangseinheit übermittelbare Daten zu speichern, insbesondere Identifikations- oder Betriebsdaten des Diagnose- oder Behandlungselements, zum Beispiel auch die von der Thermoelementverbindung ermittelten Temperaturmesswert, oder Daten über die Reinigung oder Pflege des Diagnose- oder Behandlungselements.

Eine medizinische, insbesondere dentale Behandlungsvorrichtung umfasst ein medizinisches, insbesondere dentales, Diagnose- oder Behandlungselement mit einer ersten Spule, Wicklung oder Antenne, die ein erstes Material aufweist, welches Teil einer Thermoelementverbindung zur Messung der Temperatur im Diagnose- oder Behandlungselement oder eines Bauteils des Diagnose- oder Behandlungselements ist, und eine zweite Spule, Wicklung oder Antenne, welche zur Daten- und / oder Energieübertragung berührungslos, insbesondere induktiv, mit der ersten Spule, Wicklung oder Antenne koppelbar ist, wobei die zweite Spule, Wicklung oder Antenne bevorzugt an einem mit dem Diagnose- oder Behandlungselement verbindbarem Anschlussteil, Wirkteil, Werkzeug oder Antriebsteil oder an einer von dem Diagnose- oder Behandlungselement separaten Lese- und / oder Schreibvorrichtung vorgesehen ist. Mittels dieser Behandlungsvorrichtung können somit in vorteilhafter Weise insbesondere uni- oder bidirektionale Daten zwischen den beiden Spulen übermittelt werden.

Ein bevorzugtes Verfahren zur Messung eines Temperaturmesswerts mittels eines medizinischen, insbesondere dentalen, Diagnose- oder Behandlungselements oder einer medizinischen, insbesondere dentalen Behandlungsvorrichtung ist dadurch definiert, dass mittels der Thermoelementverbindung, die ein erstes Material der ersten Spule, Wicklung oder Antenne umfasst, ein Temperaturmesswert ermittelt wird und der Temperaturmesswert oder auf Basis des Temperaturmesswerts erhaltene Temperaturdaten mittels der ersten Spule, Wicklung oder Antenne berührungslos, insbesondere induktiv, übertragen werden. Insbesondere erfolgen die Ermittlung des Temperaturmesswerts und die Übertragung des Temperaturmesswerts oder der Temperaturdaten zeitlich versetzt. Alternativ sind eine zeitgleiche Ermittlung des Temperaturmesswerts und Übertragung des Temperaturmesswerts oder der Temperaturdaten oder anderer Daten unter Verwendung eines Filters für hochfrequente und für niederfrequente Signale möglich.

Die Erfindung wird nachfolgend anhand eines bevorzugten Ausführungsbeispiels und Bezug nehmend auf die beigefügten Zeichnungen erläutert:
Figur 1 zeigt eine Ausführungsform eines medizinischen, insbesondere dentalen, Diagnose- oder Behandlungselements mit einer ersten Spule oder Wicklung, die Teil einer Thermoelementverbindung zur Messung der Temperatur im Diagnose- oder Behandlungselement oder eines Bauteils des Diagnose- oder Behandlungselements ist.
Figur 2 zeigt den in der Figur 1 mit ,D' gekennzeichneten Ausschnitt des Diagnose- oder Behandlungselements.

Die Figuren 3A und 3B zeigen zwei Ausführungsformen einer Spule oder Wicklung, die Teil einer Thermoelementverbindung sind.

Figur 3C zeigt eine weitere Ausführungsform einer ersten Spule oder Wicklung, die Teil einer Thermoelementverbindung ist, sowie eine zweite Spule oder Wicklung, welche zur Daten- und / oder Energieübertragung induktiv mit der ersten Spule koppelbar ist.

Die Figuren 3D und 3E zeigen zwei Ausführungsformen von Antennen, die Teil einer Thermoelementverbindung sind.

Die Figuren 4A und 4B zeigen zwei Ausführungsformen des Kontaktbereichs der unterschiedlichen Materialien von Thermoelementverbindungen.
Figur 5 zeigt eine Spule oder Wicklung, die Teil einer Thermoelementverbindung ist, und eine Ausführungsform einer damit verbundenen Vorrichtung zur Signalverarbeitung.
Figur 6 zeigt eine Spule oder Wicklung, die Teil einer Thermoelementverbindung ist, und eine damit verbundene Vorrichtung zur zeitgleichen Messung des Temperaturmesswerts durch die Thermoelementverbindung und zur Übertragung des Temperaturmesswerts oder darauf basierender Temperaturdaten oder anderer Daten.

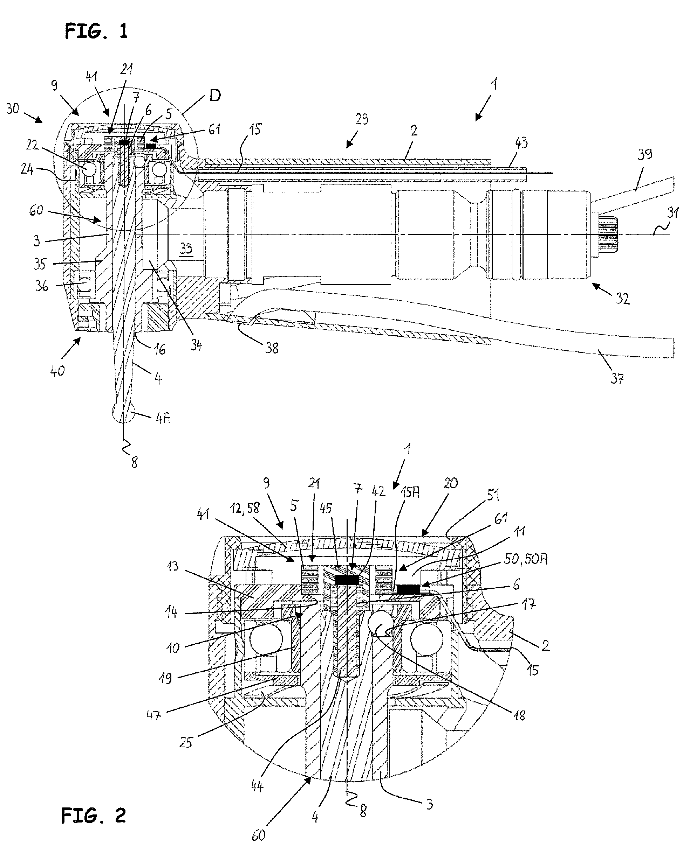

Die Figuren 1 und 2 zeigen zwei separate medizinische, insbesondere dentale Diagnose- oder Behandlungselemente, nämlich ein Handgriffelement 1 und ein damit lösbar verbundenes, durch das Handgriffelement 1 antreibbares Behandlungswerkzeug 4. Das medizinische, insbesondere dentale, Handgriffelement 1 oder Handstück weist eine Außenhülse 2 auf und besteht aus einem Griffabschnitt 29 und einem Kopfabschnitt oder Kopfteil 30. Am Kopfabschnitt 30 ist seitlich eine Werkzeugaufnahmeöffnung 16 zur Aufnahme des Behandlungswerkzeugs 4 vorgesehen, so dass das Werkzeug 4 gewinkelt zu Längsachse 31 des Griffabschnitts 29 angeordnet ist. Das Werkzeug 4 ist gemäß der in der Figur 1 dargestellten Ausführungsform als in Drehung versetzbarer Dentalbohrer ausgebildet.

Im Inneren des hohlen, länglichen Griffabschnitts 29 befinden sich Antriebsmittel 32, insbesondere eine oder mehrere Antriebswellen 33, die mit einem Motor verbindbar oder verbunden sind. Die Antriebswelle 33 weist an ihrem dem Kopfteil 30 zugewandten Ende ein Zahnrad 34 auf, das mit einem weiteren Zahnrad oder Ritzel 35 im Kopfteil 30 kämmt. Das Zahnrad 35 ist mit einer Hohlwelle 3 verbunden oder als Teil der Hohlwelle 3 ausgebildet. Die Hohlwelle 3 dient zur Aufnahme des Behandlungswerkzeugs 4, so dass die Antriebsbewegung des Motors über die die Welle 33, die Zahnräder 34, 35 und die Hohlwelle 3 umfassenden Antriebsmittel 32 auf das Behandlungswerkzeug 4 übertragbar ist. Die Antriebsmittel 32, insbesondere die Antriebswelle 33, die Zahnräder 34, 35 und die Hohlwelle 3, bilden somit eine Vorrichtung 60 zum indirekten Wirken auf ein Zielgebiet.

Die Hohlwelle 3 ist durch zwei Wälzlager, insbesondere Kugellager 22, 36, drehbar im Kopfabschnitt 30 des Handgriffelements 1 gelagert. Sie weist eine Rotations- oder Mittelachse 8, eine axiale Ausdehnung und eine radiale Ausdehnung auf. Die Hohlwelle 3 besteht zum Beispiel aus Metall, insbesondere aus Stahl, sie kann jedoch gemäß einem Ausführungsbeispiel auch aus einem elektrisch nicht leitenden, insbesondere keramischen, Material hergestellt sein, um den induktiven Energie- oder Datentransfer nicht zu beeinflussen.

Im Griffabschnitt 29 sind des Weiteren eine oder mehrere Medienleitungen vorgesehen, zum Beispiel ein optischer Leiter 37, insbesondere ein Glasfaserstab, der Strahlung einer Strahlungsquelle zu einem Lichtabgabefenster 38 leitet, und eine oder mehrere Fluidleitungen 39, insbesondere zur Leitung von Behandlungsfluiden wie Druckluft und / oder Wasser. Die zumindest eine Fluidleitung 39 ist mit einer oder mehreren Fluidquellen und mit einer Fluidabgabevorrichtung 40 verbunden, die zum Beispiel eine oder mehrere Fluidabgabeöffnungen, eine Mischkammer oder eine Sprayplatte umfasst. Die Fluidabgabevorrichtung ist um die Werkzeugaufnahmeöffnung 16 angeordnet.

Im Kopfteil 30 des Handgriffelements 1 sind des Weiteren eine Werkzeughalte-/Lösevorrichtung 9 zum Befestigen des Werkzeugs 4 in der Hohlwelle 3 und zum Lösen des Werkzeugs 4 aus der Hohlwelle 3 und eine erste Spule oder Wicklung 5 angeordnet. Die gesamte Werkzeughalte-/Lösevorrichtung 9 befindet sich an dem der Werkzeugaufnahmeöffnung 16 abgewandten Ende der Hohlwelle 3. Die ebenfalls an dem der Werkzeugaufnahmeöffnung 16 abgewandten Ende der Hohlwelle 3 angeordnete erste Spule 5 ist in einer Aufnahme oder in einem Hohlraum 11 innerhalb der Werkzeughalte-/Lösevorrichtung 9 oder in einem durch die Werkzeughalte-/Lösevorrichtung 9 gebildeten Hohlraum 11 aufgenommen. Bevorzugt ist das Volumen des Hohlraums 11 größer bemessen als das Volumen oder der Außenumfang der ersten Spule 5, so dass der Hohlraum 11, insbesondere jener Teil des Hohlraums 11, der mit Luft gefüllt ist, auch Teil einer elektrischen Isolierschicht ist, die die erste Spule 5 umgibt. Die Spule 5 ist Teil einer Sende- und / oder Empfangseinheit 61 zur berührungslosen Daten- und / oder Energieübertragung des Handgriffelements 1.

Die erste Spule 5 oder die Sende- und / oder Empfangseinheit 61 ist Teil einer Daten- und/oder Energietransfereinheit 41, die gemäß den Figuren 1 und 2 zum Beispiel auch eine am Werkzeug 4 vorgesehene Speichereinheit 7 umfasst. Die Speichereinheit 7 weist eine zweite Spule oder Wicklung 6 und einen Speicher 42 auf, wobei der Speicher 42 entweder als Lesespeicher (ROM) oder als Lese- und Schreibspeicher (RAM) ausgebildet ist. Die beiden Spulen 5, 6 sind berührungslos, insbesondere induktiv, miteinander koppelbar, so dass Energie und / oder Daten zwischen den Spulen 5, 6 übertragen werden können. Dazu ist die erste Spule 5 über eine oder mehrere, zum Beispiel zwei, elektrische Leitungen 15 mit einer innerhalb oder außerhalb des Handgriffelements 1 angeordneten Energiequelle verbindbar oder verbunden. Die von der Energiequelle zur Verfügung gestellte elektrische Energie wird induktiv von der ersten Spule 5 auf die zweite Spule 6 und weiter zu dem Speicher 42 übertragen. Durch diese Energieversorgung können der Speicher 42 bzw. ein den Speicher 42 umfassender elektronischer Chip aktiviert und Speicherdaten, zum Beispiel Identifikationsdaten oder Betriebsdaten des Werkzeugs 4, vom Speicher 42 gelesen werden bzw. Speicherdaten, zum Beispiel Daten über die Betriebsdauer, den Reinheits- oder Sterilitätszustand oder über den Verschleiß des Werkzeugs 4, auf den Speicher 42 geschrieben werden. Die Übertragung der Speicherdaten erfolgt ebenfalls induktiv über die beiden Spulen 5, 6, die nun auch als Antennen dienen und eine, bevorzugt hochfrequente, Datenübertragung, insbesondere im Radiofrequenzbereich, gewährleisten. Demgemäß umfasst die Daten- und/oder Energietransfereinheit 41 bzw. die Speichereinheit 7 bevorzugt ein RFID-Chip oder RFID-Etikett oder einen RFID-Transponder. Die Leitung der Speicherdaten vom Speicher 42 bzw. gegebenenfalls zum Speicher 42 erfolgt ebenfalls über die zumindest zwei elektrischen Leitungen 15.

Das Handgriffelement 1 ist des Weiteren mit einer Speichereinheit 50 versehen, die ein Speicherelement zur Speicherung von Betriebs- und / oder Identifikationsdaten des Handgriffelements 1 und eine mit dem Speicherelement verbundene Lese- und/ oder Schreibvorrichtung 50A zum Auslesen von und/ oder Beschreiben des Speicherelements mit Betriebs- und / oder Identifikationsdaten umfasst. Die Speichereinheit 50 ist über die zwei elektrischen Leitungen 15 mit einer Kupplungsvorrichtung des Handgriffelements 1 verbunden, über die eine Verbindung zu einer Steuer- und / oder Versorgungseinheit des Handgriffelements 1 herstellbar ist. Zwei elektrische Leitungen 15A verbinden die Speichereinheit 50 mit dem Speicherelement und der Lese- und/ oder Schreibvorrichtung 50A mit der ersten Spule 5.

Zum Auslesen von Daten aus dem Speicherelement der Speichereinheit 50 oder zum Speichern von Daten auf dem Speicherelement sowie zur Übertragung von Versorgungsenergie stehen dem Anwender somit zwei Wege frei: Der Energie- und / oder Datentransfer kann entweder leitungsgebunden über die elektrischen Leitungen 15, 15A erfolgen, die Energie, insbesondere elektrische Energie, von der Steuer- und / oder Versorgungseinheit oder direkt von einer Energiequelle erhalten und / oder die mit einer Steuerung, insbesondere einem Mikrocomputer, der Steuer- und / oder Versorgungseinheit verbunden oder verbindbar sind und die Daten der Steuerung zuführen bzw. von der Steuerung erhalten.

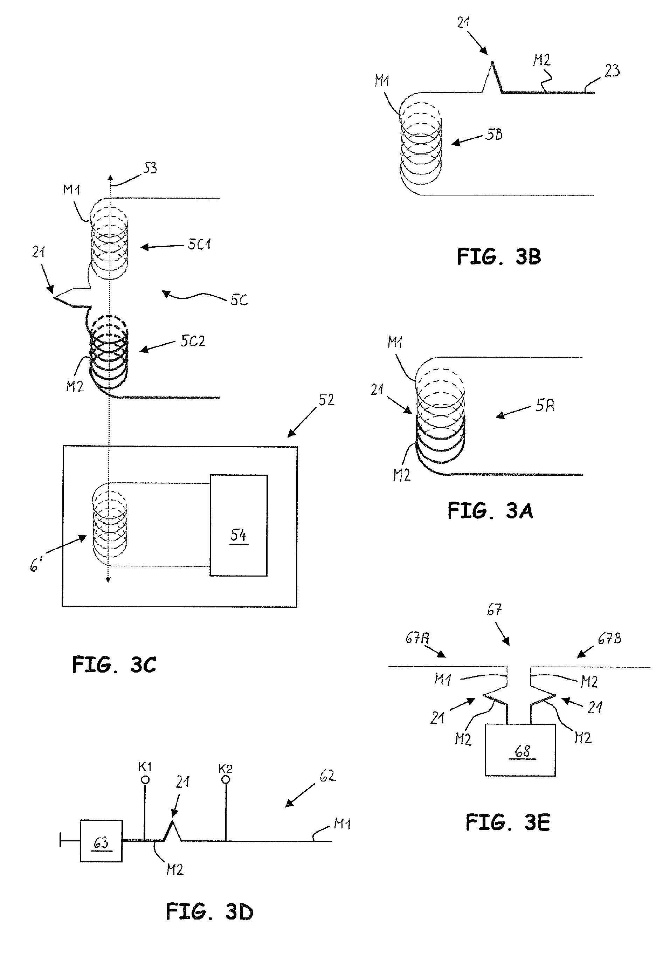

Alternativ erfolgt der Energie- und / oder Datentransfer von oder zur Speichereinheit 50 berührungslos, insbesondere induktiv, über die erste Spule 5. Dazu muss das Handgriffelement 1 derart an ein Lese- und / oder Sendegerät 52 (siehe Figur 3C) angenähert werden, dass eine induktive Kopplung der ersten Spule 5 mit einer weiteren Spule 6' des Lese- und / oder Sendegeräts 52 entsteht.

Selbstverständlich ist es auch möglich zur Energie- und / oder Datenübertragung beide Übertragungswege gleichzeitig zu nutzen: So kann zum Beispiel die Speichereinheit 50 über die elektrischen Leitungen 15, 15A mit Energie versorgt werden, während gleichzeitig ein induktiver Datentransfer zwischen dem Lese- und / oder Sendegerät 52 und dem Handgriffelement 1 erfolgt. Alternativ kann ein induktiver Energietransfer zwischen dem Lese- und / oder Sendegerät 52 und dem Handgriffelement 1 stattfinden und ein leitungsgebundener Datenaustausch über die Leitung 15, die gemäß diesem Ausführungsbeispiel auch als optischer Leiter ausgebildet sein kann.

Die zwei elektrischen Leitungen 15 verlaufen im Inneren des Handgriffelements 1 und sind durch Isoliermittel, zum Beispiel eine Kunststoffhülle, elektrisch von metallischen Bauteilen des Handgriffelements 1, insbesondere von der Außenhülse 2, isoliert. Des Weiteren sind die Leitungen 15 von einem Kunststoff- oder Metallrohr 43 umgeben, das zur Abschirmung der Leitungen 15 vor externen Störfeldern dient, zum Beispiel von Magnetfeldern oder elektrischen Feldern, die insbesondere von einem das Werkzeug 4 antreibenden Elektromotor stammen.

Zur Weiterverarbeitung der vom Speicher 42 erhaltenen Speicherdaten und / oder zur Abgabe von Speicherdaten auf den Speicher 42 ist innerhalb oder außerhalb des Handgriffelements 1 eine Steuereinheit, insbesondere ein Mikrocomputer, vorgesehen, der über die zumindest zwei elektrischen Leitungen 15 mit dem Speicher 42 verbunden ist. Bevorzugt erhält die Steuereinheit Identifikationsdaten vom Speicher 42, die das in die Hohlwelle 3 des Handgriffelements 1 eingesetzte und mit dem Speicher 42 verbundene Werkzeug 4 identifizieren und die zur Ansteuerung des das Handgriffelement 1 antreibenden Motors oder der Kühlmittelversorgung dienen. Zum Beispiel werden auf diese Weise das Drehmoment oder die Drehzahl des Motors oder die dem Werkzeug 4 zugeleitete Kühlmittelmenge auf maximale Werte begrenzt, die den Eigenschaften des Werkzeugs oder den Anforderungen der medizinischen Behandlung, die mit dem Werkzeug 4 durchführbar ist, entsprechen. Die Steuereinheit bildet somit gemäß diesem besonders bevorzugten Ausführungsbeispiel gemeinsam mit den elektrischen Leitungen 15 und der Daten- und/oder Energietransfereinheit 41 eine Werkzeugerkennungsvorrichtung.

Der detaillierte Aufbau der Daten- und/oder Energietransfereinheit 41 ist insbesondere aus der Figur 2 erkennbar. Die, bevorzugt unlösbar, mit dem Werkzeug 4 verbundene Speichereinheit 7 umfasst die zweite Spule 6, den Speicher 42 und einen, insbesondere stabförmigen, Ferritkern 44. Die Speichereinheit 7 ist an und / oder auf dem dem Behandlungsabschnitt 4A (siehe Figur 1) gegenüberliegenden Ende des Werkzeugs 4 angeordnet, wobei Teile der Speichereinheit 7, insbesondere der Ferritkern 44, im Werkzeugschaft bzw. in einer Aufnahme des Werkzeugschafts versenkt sind. Alternativ ist die gesamte Speichereinheit 7 auf das Ende des Werkzeugschafts aufgesetzt, ohne dass dabei Teile der Speichereinheit 7 in den Werkzeugschaft ragen. Befestigungsmittel 45, zum Beispiel ein nicht leitendes Isoliermaterial, bevorzugt ein Vergussharz, insbesondere Epoxidharz, befestigen die Speichereinheit 7 am Werkzeug 4. Bevorzugt ist der elektronische Speicher 42 Teil eines elektronischen Chips, das zusätzlich einen Mikrocomputer oder Schaltkreis umfasst, zum Beispiel zum Lesen der Daten aus dem Speicher 42, zur Energieversorgung des Speichers 42 oder gegebenenfalls zum Schreiben von Daten auf den Speicher 42.

Die Daten- und/oder Energietransfereinheit 41 umfasst, wie im Vorstehenden bereits erwähnt, auch die im Handgriffelement 1 in Bezug auf die Hohlwelle 3 unbeweglich angeordnete erste Spule 5 bzw. die Sende- und / oder Empfangseinheit 61. Die erste Spule 5 ist auf einem Träger 13 gelagert, zum Beispiel auf einem flächigen, insbesondere platten- oder scheibenförmigen, unbeweglich in Handgriffelement 1 bzw. unbeweglich in Bezug auf die Hohlwelle 3 angeordneten Träger 13. Der Träger 13 weist eine Öffnung 14 auf, um die die erste Spule 5 angeordnet ist und die derart bemessen ist, dass sie für die Speichereinheit 7 des Behandlungswerkzeugs 4 zumindest teilweise durchsetzbar ist, so dass die erste Spule 5 und die Speichereinheit 7 des Behandlungswerkzeugs 4, insbesondere deren zweite Spule 6, ineinander anordenbar sind und ein induktiver Energie- und / oder Datentransfer zwischen den beiden Spulen 5, 6 möglich ist oder begünstigt wird. Ein oder mehrere Rücksprünge oder Vertiefungen am Träger 13 dienen zur Aufnahme oder zum Stützen der ersten Spule 5 und der mit der ersten Spule 5 verbundenen elektrischen Leitungen 15. Der Träger 13, der Teil einer die erste Spule 5 umgebenden elektrischen Isolierschicht ist, umfasst bevorzugt ein elektrisch isolierendes Material, insbesondere Kunststoff, Glas oder Keramik.

Die erste Spule 5 ist gemäß einem Ausführungsbeispiel an dem der Werkzeughalte-/Lösevorrichtung 9 des Handgriffelements 1 zugewandten Ende 10 der Hohlwelle 3 außerhalb der axialen Ausdehnung und innerhalb der radialen Ausdehnung der Hohlwelle 3 angeordnet. Die Öffnung 14 des Trägers 13 und die erste Spule 5 sind konzentrisch zur Mittelachse 8 der Hohlwelle 3 angeordnet. Selbstverständlich ist es jedoch auch möglich die Spule oder Wicklung 5 an einer anderen Position im Handgriffelement 1, insbesondere im Kopfabschnitt 30, anzuordnen.

Aus der Figur 2 ist des Weiteren der detaillierte Aufbau der Werkzeughalte-/Lösevorrichtung 9 zu erkennen. Die Werkzeughalte-/Lösevorrichtung 9 ist an dem der Werkzeugaufnahmeöffnung 16 gegenüber liegenden Ende 10 der Hohlwelle 3 angeordnet bzw. umgibt dieses Ende 10. Die Werkzeughalte-/Lösevorrichtung 9 umfasst unter anderem ein eine Bohrung 17 der Hohlwelle 3 durchsetzendes und durch die Bohrung 17 in das Innere der Hohlwelle 3 ragendes Formelement 18, eine beweglich angeordnete, mit dem Formelement 18 zusammenwirkende Verriegelungshülse 19 und ein Betätigungselement 20 zum Bewegen der Verriegelungshülse 19. Das Formelement 18 ist zum Beispiel als Kugel, Halbkugel, Ellipsoid oder als Zylinder ausgebildet. Um eine gleichmäßige und sichere Verspannung des Werkzeugs 4 in der Hohlwelle 3 zu erreichen, sind bevorzugt mehrere Formelemente 18, zum Beispiel drei oder sechs Formelemente 18, insbesondere gleichmäßig, um die Hohlwelle 3 angeordnet.

Die Verriegelungshülse 19 umgibt die Hohlwelle 3 und ist axial, entlang der Mittelachse 8 der Hohlwelle 3 verschiebbar. Die Verriegelungshülse 19 weist einen ersten Abschnitt mit einem ersten Innendurchmesser und einen zweiten Abschnitt mit einem zweiten, größeren Innendurchmesser auf, wobei der zweite Abschnitt von dem Außenmantel der Hohlwelle 3 beabstandet ist. Die beiden Abschnitte sind durch einen kurzen konischen Abschnitt miteinander verbunden. Wird die Verriegelungshülse 19 in eine Position verschoben in der, wie in Figur 2 dargestellt, der erste Abschnitt das Formelement 18 berührt und derart weit in die Bohrung 17 drückt, dass ein Teil des Formelements 18 in das Innere der Hohlwelle 3 ragt, so kontaktiert das Formelement 18 das in die Hohlwelle 3 eingesetzte Werkzeug 4, insbesondere in einer oder mehreren Vertiefungen am Werkzeugschaft, wodurch das Werkzeug 4 in der Hohlwelle 3 axial und zur Übertragung des Drehmoments auf das Werkzeug 4 befestigt ist. Wird die Verriegelungshülse 19 in eine Position verschoben, in der der zweite Abschnitt der Verriegelungshülse 19 das Formelement 18 umgibt, so kann das Formelement 18, bezogen auf die Mittelachse 8, radial nach außen weichen, wodurch es nicht mehr in die Vertiefung am Werkzeugschaft oder in das Innere der Hohlwelle 3 ragt und das Werkzeug 4 zum Entnehmen aus der Hohlwelle 3 frei gibt.

Zumindest ein Teil der Verriegelungshülse 19 umgibt den Außenmantel der Hohlwelle 3 und liegt gemäß einem Ausführungsbeispiel am Innenlaufring des Kugellagers 22 an, so dass die Verriegelungshülse 19 gemeinsam mit der Hohlwelle 3 rotiert. Unterhalb des Kugellagers 22 befinden sich eine ringförmige Lagerscheibe 47 und ein Federelement 25. Das Federelement 25, das insbesondere als Blattfeder ausgebildet ist, spannt die Verriegelungshülse 19 in der in Figur 2 dargestellten Verriegelungsposition vor, in der das Werkzeug 4 in der Hohlwelle 3 fixiert ist. Zum Lösen des Werkzeugs 4 aus der Hohlwelle muss der Anwender die Federkraft des Federelements 25 überwinden, um die Verriegelungshülse 19 in Richtung der Werkzeugaufnahmeöffnung 16 zu verschieben. Da die Verriegelungshülse 19 mit dem Kugellager 22 verbunden ist, sind das Kugellager 22 und die Lagerscheibe 47 gemäß dem in der Figur 2 dargestellten Ausführungsbeispiel ebenfalls verschiebbar im Handgriffelement 1 angeordnet.

Die Verriegelungshülse 19, das Kugellager 22, das Federelement 25 und die Lagerscheibe 47 sind in einem Käfig 24 aufgenommen, der die Hohlwelle 3 umgibt und der unbeweglich, insbesondere undrehbar, im Handgriffelement 1 aufgenommen ist. Der Käfig 24 weist zum Beispiel eine die Hohlwelle 3 konzentrisch umgebende, zylindrische Außenwand und eine Bodenplatte mit einer Öffnung zum Durchtritt der Hohlwelle 3 auf.

Das Betätigungselement 20 der Werkzeughalte-/Lösevorrichtung 9 umfasst gemäß den Figuren 1 und 2 einen Druckknopf oder Druckdeckel 12. Der Druckdeckel 12 ist zumindest teilweise oder vollständig in der Außenhülse 2 des Handgriffelements 1 versenkt und mittels ein oder mehrerer Führungselemente entlang der Mittelachse 8 der Hohlwelle 3 verschiebbar. Das Betätigungselement 20 oder der Druckdeckel 12 umfassen eine eine Öffnung 51 verschließende, insbesondere gewölbte, Kappe 58 und zwei Kontaktelemente. Die zwei Kontaktelemente sind am unmittelbaren Außenrand der Kappe 58 und somit radial beabstandet von der Verriegelungshülse 19 oder der Hohlwelle 3 angeordnet. Durch das Ausüben von Druck auf den Druckdeckel 12 wird dieser mit den beiden Kontaktelementen in Richtung der Verriegelungshülse 19 verschoben, bis die Kontaktelemente die Verriegelungshülse 19 kontaktieren und ebenfalls verschieben, wodurch das Werkzeug 4 aus der Hohlwelle gelöst oder in der Hohlwelle 3 fixiert werden kann.

Durch die seitliche oder radial nach außen verschobene Anordnung der Kontaktelemente im Kopfteil 30 des Handgriffelements 1 und / oder durch die Anordnung der Kontaktelemente am Außenrand der Kappe 58 des Betätigungselements 20, und bevorzugt zusätzlich durch die Wölbung der Kappe 58, entsteht im Kopfteil 30 oder innerhalb der Werkzeughalte-/Lösevorrichtung 9 eine Aufnahme oder ein Hohlraum 11, in dem in vorteilhafter Weise die erste Spule 5 und bevorzugt auch der Träger 13 und die Speichereinheit 7 der Werkzeugs 4 aufnehmbar sind. Insbesondere ist der Hohlraum 11 zwischen dem der Werkzeughalte-/Lösevorrichtung 9 des Handgriffelements 1 zugewandten Ende 10 der Hohlwelle 3 und dem Druckdeckel 12 der Werkzeughalte-/Lösevorrichtung 9 angeordnet. Der Träger 13 und die erste Spule 5 sind somit innerhalb der Werkzeughalte-/Lösevorrichtung 9 aufgenommen bzw. es sind an beiden Seiten des Trägers 13 Teile der Werkzeughalte-/Lösevorrichtung 9 angeordnet, wodurch insbesondere die erste Spule 5 geschützt ist und zusätzlich ein besonders kompakter Aufbau des Kopfteils 30 erzielt wird.

Erfindungsgemäß dienen die Spulen 5, 6 jedoch nicht nur als Teil der Sende- und / oder Empfangseinheit 61 oder als Teil der Daten- und/ oder Energietransfereinheit 41 zum berührungslosen, insbesondere induktiven, Daten- und / oder Energietransfer, sondern zumindest eine der Spulen 5, 6 ist auch Teil einer Thermoelementverbindung 21 oder bildet eine Thermoelementverbindung 21. Die Thermoelementverbindung 21 dient zum Ermitteln von Temperaturmesswerten des Handgriffelements 1, bevorzugt der Außenhülse 2 oder des Kopfabschnitts 30 oder Teilen davon, besonders bevorzugt der Werkzeughalte-/Lösevorrichtung 9, des Betätigungselements 20, des Druckdeckels 12, zumindest eines Lagers 22, 36 oder anderer bewegter Bauteile oder bewegte Bauteile kontaktierender Bauteile des Handgriffelements 1 oder des Kopfabschnitts 30.

Gemäß bevorzugten Ausführungsbeispielen werden jedoch nicht nur die Spulen 5, 6 zur Ermittlung von Temperaturmesswerten verwendet, sondern es werden noch weitere, mit den Spulen 5, 6 verbundene Bauteile des Diagnose- oder Behandlungselements zum Beispiel für die Übertragung, Leitung, Auswertung, den Empfang oder die Sendung der Temperaturmesswerten oder davon abgeleiteter Daten verwendet. So können insbesondere die elektrischen Leitungen 15, 15A zum Leiten oder Übertragen der Werte/Daten, die Speichereinheiten 7, 50 zum Speichern oder Lesen der Werte/Daten, Schaltkreise oder Mikrokontroller des Diagnose- oder Behandlungselements zum Verarbeiten der Werte/Daten und selbstverständlich die Spulen 5, 6 bzw. die Daten- und / oder Energietransfereinheit 41 bzw. die Sende- und / oder Empfangseinheit 61 zum induktiven Übertragen der Werte/Daten genutzt werden. Entsprechend wird bei einem bevorzugten Betriebsverfahren des Diagnose- oder Behandlungselements zuerst ein Temperaturmesswert durch die Spule 5, 6 ermittelt und dieser nachfolgend von der Spule 5, 6 berührungslos, insbesondere induktiv, übertragen.

Die Figuren 3A ― 3C zeigen unterschiedliche Ausführungsformen von Spulen oder Wicklungen 5A ― 5C, die für die Ermittlung von Temperaturmesswerten und für die induktive Daten- und / oder Energieübertragung geeignet sind und dem gemäß dafür an dem Handgriffelement 1 oder an dem Werkzeug 4 verwendet werden können. Die Spulen 5A ― 5C sind aus elektrisch leitendem oder metallischem Spulendraht gefertigt.

Die Spule 5A der Figur 3A weist ein erstes elektrisch leitendes Material M1 und ein zweites elektrisch leitendes Material M2 auf, so dass die Thermoelementverbindung 21 durch die erste Spule oder Wicklung 5A gebildet ist.

Die Spule oder Wicklung 5B der Figur 3B weist ein erstes elektrisch leitendes Material M1 auf. Ein mit der Spule 5B verbundener elektrischer Leiter 23 weist ein zweites Material M2 auf, so dass die Thermoelementverbindung 21 durch die erste Spule 5B und den damit verbundenen elektrischen Leiter 23 gebildet ist.

Die Spule oder Wicklung 5C der Figur 3C ist durch zwei Teilspulen oder Teilwicklungen 5C1 und 5C2 gebildet, wobei die Teilspule 5C1 ein erstes Material M1 und die Teilspule 5C2 ein zweites Material M2 aufweist. Die beiden Teilspulen 5C1, 5C2 sind an jeweils einem freien Ende miteinander verbunden, wobei die beiden freien Enden gemäß dem in der Figur 3C dargestellten Ausführungsbeispiel nicht spiralig ausgebildet sind, sondern insbesondere durch kurze, gerade Leiterabschnitte gebildet sind. Die freien, miteinander verbundenen Enden können jedoch selbstverständlich auch andere Formen oder Geometrien aufweisen. Der Vorteil der insbesondere nicht spiraligen oder nicht gewundenen Ausbildung der Kontaktstelle oder Thermokontaktstelle der beiden Materialien M1, M2, so wie sie in den Figuren 3B und 3C dargestellt ist, liegt insbesondere darin, dass die Kontaktstelle oder Thermokontaktstelle damit in (besonders kleine) Zwischenräume passt, in welchen die gesamte Spule 5C oder die Teilwicklungen 5C1 und 5C2 keinen Platz finden.

Die Figur 3D zeigt eine Antenne 62, insbesondere eine Stabantenne, die mit einer Quelle 63, insbesondere eine Hochfrequenzquelle, verbunden ist. Die Antenne 62 weist ein erstes Material M1 und ein zweites Material M2 auf, welches als Verbindung zur Quelle 63 dient. Die beiden Materialien M1, M2 sind wiederum Teil einer Thermoelementverbindung 21 zur Bestimmung eines Temperaturmesswertes. Die sich aufgrund der Temperaturdifferenz ergebende elektrische Thermospannung der Materialien M1, M2 ist zwischen den Kontaktstellen K1 / K2 messbar.

Die Figur 3E zeigt eine dipolartige Antenne oder Dipolantenne 67 mit zwei Antennenarmen 67A, 67B und einer Sender-/Empfängereinheit 68. Zumindest einer der beiden Antennenarme 67A, 67B weist ein erstes Material M1 und ein zweites Material M2 auf, welches Teil einer Thermoelementverbindung 21 zur Bestimmung eines Temperaturmesswertes ist. Bevorzugt weisen beide Antennenarme 67A, 67B ein erstes Material M1 und ein zweites Material M2 auf, welches Teil einer Thermoelementverbindung 21 ist, so dass in vorteilhafter Weise mittels einer derartigen Dipolantenne 67 Temperaturmesswerte an zwei unterschiedlichen Positionen messbar sind.

Für alle Ausführungsformen der Figuren 3A ― 3E gilt, dass die beiden Materialien M1 und M2 unterschiedliche Materialien sind, insbesondere unterschiedliche Metalle, so dass eine bimetallische Thermoelementverbindung 21 vorliegt. Bevorzugt umfasst eines der beiden Materialien M1, M2 Kupfer oder eine Legierung mit einem hohen Kupferanteil und das andere der beiden Materialien M1, M2 ein anderes Metall oder eine andere Legierung mit Nickel, Chrom oder Eisen, insbesondere Konstantan.

In der Figur 3C ist zusätzlich eine Lese- und / oder Sendevorrichtung 52 schematisch dargestellt, welche eine zweite Spule oder Wicklung 6' umfasst. Zwecks Daten- und / oder Energieübertragung sind die beiden Spulen 5C, 6' induktiv miteinander koppelbar, dies ist durch den Pfeil 53 angedeutet. Selbstverständlich sind die beiden Spulen 5A, 5B in gleicher Weise mit der Spule 6' induktiv koppelbar. An die Spule 6' ist eine Auswerteeinheit 54 oder eine Steuer- und / oder Regeleinheit, insbesondere mit einem Mikrokontroller, angeschlossen, welche Energie für die Energieübertragung zur Verfügung stellt und / oder Daten für die Datenübertragung zur Verfügung stellt und / oder empfängt und / oder auswertet. Bevorzugt werden mittels der Daten- und / oder Energieübertragung die von der Thermoelementverbindung 21 ermittelten Temperaturmesswerte übertragen. Die von den Spulen 5A ― 5C wegführenden elektrischen Leitungen (zum Beispiel Leiter 23 in Figur 3B) können ebenfalls zur Daten- und / oder Energieübertragung zu den Spulen 5A ― 5C oder von den Spulen 5A ― 5C verwendet werden.

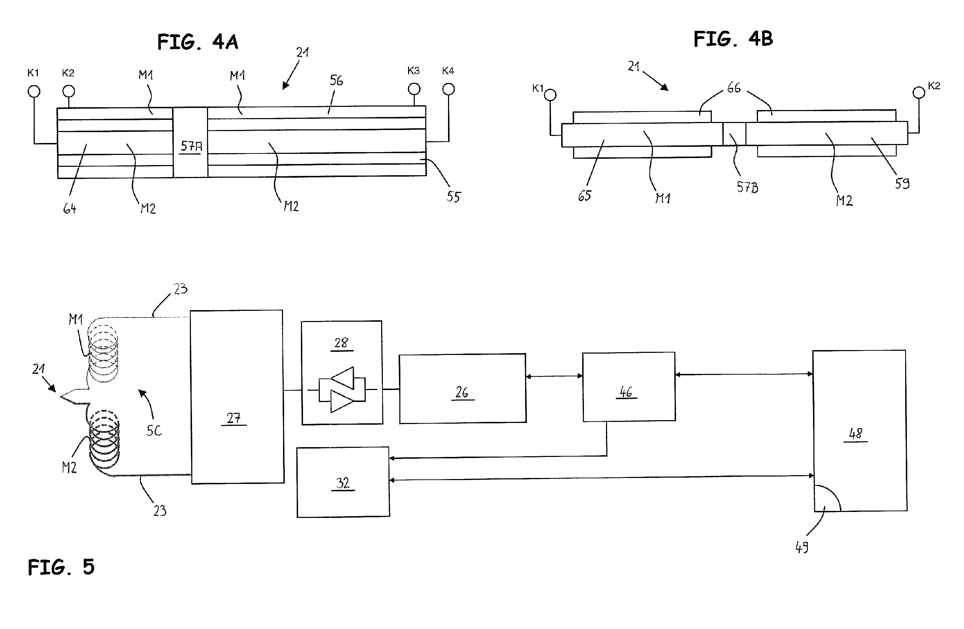

Die Figuren 4A und 4B zeigen zwei Ausführungsbeispiele von Kontaktbereichen, Kontaktstellen oder Thermokontaktstellen der Materialien M1 und M2 der Thermoelementverbindung 21. In der Figur 4A ist das Material M2 als, insbesondere zylindrischer und im Querschnitt kreisförmiger, Draht 64 ausgebildet. Der Draht 64 ist abgesehen von einem kurzen Abschnitt von einem elektrisch isolierenden Material, zum Beispiel Kunststoff umgeben. Das isolierende Material ist bevorzugt als hohle Hülse 55 ausgebildet, in welcher der Draht 64 aufgenommen ist. Um die Hülse 55 ist das Material M1 angeordnet, welches bevorzugt wiederum als hohlzylindrische Hülse 56 ausgebildet ist, in welcher das isolierende Material und das Material M2 aufgenommen sind. An der Thermokontaktstelle 57A, an der die beiden Materialien M1 und M2 direkt miteinander verbunden sind, erfolgt die Bestimmung des Temperaturmesswertes. Die sich aufgrund der Temperaturdifferenz ergebende (elektrische) Thermospannung der Materialien M1, M2 ist wahlweise zwischen den Kontaktstellen K1 / K2 und K3 / K4 oder zwischen den Kontaktstellen K1 / K3 und K2 / K4 messbar.

Gemäß der Figur 4B sind die beiden Materialien M1 und M2 als Drähte 59, 65 ausgebildet, die einander mit jeweils einem freien Enden kontaktieren, so dass die freien Enden die Thermokontaktstelle 57B bilden, an der die Bestimmung des Temperaturmesswertes erfolgt. Abhängig von der Einbausituation in dem Diagnose- oder Behandlungselement 1, 4 ist zumindest eines der Materialien M1 und M2 von elektrisch isolierenden Material 66 umgeben oder weist keines der Materialien M1 und M2 ein isolierendes Material 66 auf. Die sich aufgrund der Temperaturdifferenz ergebende (elektrische) Thermospannung der Materialien M1, M2 ist zwischen den Kontaktstellen K1 / K2 messbar.

In der Figur 5 ist die Spule oder Wicklung 5C der Figur 3C und eine damit verbundene Vorrichtung zur Signalverarbeitung dargestellt: Die Spule 5C ist mittels der elektrischen Leiter 23 mit einem Anpassnetzwerk 27, insbesondere einem Filter, verbunden, das die von der Thermoelementverbindung 21 erhaltenen Temperaturmesswerte filtert. An den Filter 27 schließt ein Verstärker 28 zur Verstärkung der Temperaturmesswerte an. Die Temperaturmesswerte gelangen schließlich zu einer Auswerteschaltung 46, welche die Temperaturmesswerte auswertet oder verarbeitet. Die derart ausgewerteten oder verarbeiteten Temperaturmesswerte können schließlich zu einer Steuer- und / oder Regelvorrichtung 48 übertragen werden, die ausgebildet ist, den Temperaturmesswert zu speichern und / oder mit einem Temperaturgrenzwert zu vergleichen und bei Überschreiten des Grenzwerts zum Beispiel eine, insbesondere optische oder akustische, Anzeigevorrichtung 49 zu aktivieren oder auf Antriebsmittel 32 des Diagnose- oder Behandlungselement 1, 4 einzuwirken, insbesondere diese zu bremsen oder anzuhalten, oder das Einwirken der Vorrichtung 60, 4A zum direkten oder indirekten Wirken auf ein Zielgebiet zu unterbrechen. Alternativ werden der Vergleich des Temperaturmesswerts mit einem Temperaturgrenzwert und die gegebenenfalls daraus resultierenden, im Vorstehenden angeführten Handlungen durch die Auswerteschaltung 46 durchgeführt oder ausgelöst oder erfolgt die Auswertung oder Verarbeitung der Temperaturmesswerte anstatt durch die Auswerteschaltung 46 durch die Steuer- und / oder Regelvorrichtung 48.

Vorzugsweise wird zumindest eines der vorgenannten Bauteile 23, 27, 28, 46, 48 nicht nur für die von der Thermoelementverbindung 21 ermittelten Temperaturmesswerte verwendet, sondern auch für die Daten und / oder Energie der berührungslosen Daten- und / oder Energieübertragung. Das Diagnose- oder Behandlungselement 1, 4 umfasst damit zumindest ein gemeinsames Verarbeitungs- und / oder Auswerteelement 23, 27, 28, 46, 48 für die Temperaturmesswerte und die Daten und / oder Energie der berührungslosen Daten- und / oder Energieübertragung. Das zumindest eine gemeinsame Verarbeitungs- und / oder Auswerteelement 23, 27, 28, 46, 48 ist vorzugsweise für eine bidirektionalen Funktion einsetzbar, so dass Daten und / oder Energie sowohl von der Spule 5C in Richtung oder bis zur Auswerteschaltung 46 oder Steuer- und / oder Regelvorrichtung 48 als auch in umgekehrter Richtung bis zur Spule 5C übertragbar sind.

Ist ein derartiges gemeinsames Verarbeitungs- und / oder Auswerteelement 23, 27, 28, 46, 48 vorhanden, so ist vorzugsweise auch eine Schaltvorrichtung 26 zur wahlweisen Übertragung und / oder Verarbeitung des von der Thermoelementverbindung 21 ermittelten Temperaturmesswerts und der Daten und / oder Energie der berührungslosen Daten- und / oder Energieübertragung vorzusehen. Die Schaltvorrichtung 26 ist zum Beispiel derart ausgebildet, dass sie wahlweise oder zeitlich versetzt Temperaturmesswerte und Daten und / oder Energie der berührungslosen Daten- und / oder Energieübertragung überträgt oder weiterleitet.

Gemäß einem Ausführungsbeispiel sind die Auswerteschaltung 46 oder die Steuer- und / oder Regelvorrichtung 48 ausgebildet, den Temperaturverlauf zumindest eines Teils einer Diagnose oder Behandlung mit dem Diagnose- oder Behandlungselement 1, 4 zu speichern oder zeitgleich oder zeitversetzt anzuzeigen oder zu übertragen.

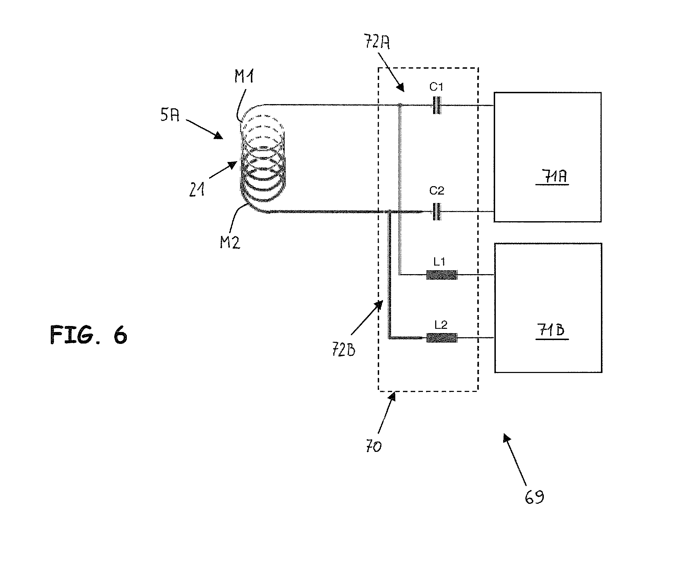

Die Figur 6 zeigt eine Spule oder Wicklung 5A, wie sie unter Bezug auf die Figur 3A beschrieben ist. Die Spule 5A ist mit einer Vorrichtung 69 verbunden oder Teil einer Vorrichtung 69, welche zur zeitgleichen Ermittlung des Temperaturmesswerts durch die Thermoelementverbindung 21 und berührungslosen, insbesondere induktiven, Übertragung des Temperaturmesswerts oder darauf basierender Temperaturdaten oder anderer Daten, insbesondere von Identifikationsdaten, Betriebsdaten oder Daten über die Reinigung oder Pflege des Diagnose- oder Behandlungselements 1, 4, oder von Energie durch die Spule 5A ausgebildet ist. Die Vorrichtung 69 umfasst einen mit der Spule 5A verbundenen Filter oder Signalteiler (Signalsplitter, Signalweiche) 70, welcher ausgebildet ist, die überlagerten Signale der berührungslosen Daten- und / oder Energieübertragung und der Temperaturmessung (die Temperaturmesswerte) zu trennen und insbesondere unterschiedlichen Verarbeitungseinheiten 71A, 71B, zum Beispiel unterschiedlichen Schaltkreisen, Steuer- oder Regeleinheiten oder Speicherelementen, zuzuführen. Der Signalteiler 70 ist insbesondere dazu ausgebildet, Gleichspannungssignale, d.h. die Signale der Temperaturmessung durch die Thermoelementverbindung 21, und Wechselspannungssignale, d.h. die Signale der berührungslosen, insbesondere induktiven Daten- und / oder Energieübertragung, zu trennen.

Gemäß dem in der Figur 6 dargestellten Ausführungsbeispiel umfasst der Signalteiler 70 einen ersten, durch zwei elektrische Leitungen gebildeten Pfad 72A, der die Spule 5A mit der Verarbeitungseinheit 71 A verbindet und die Wechselspannungssignale, d.h. die Signale der berührungslosen, insbesondere induktiven Daten- und / oder Energieübertragung, zwischen der Spule 5A und der Verarbeitungseinheit 71 A leitet. Die Verarbeitungseinheit 71 A ist demgemäß zur Verarbeitung der, insbesondere hochfrequenten, Wechselspannungssignale ausgebildet. Des Weiteren umfasst der Signalteiler 70 einen zweiten, durch zwei elektrische Leitungen gebildeten Pfad 72B, der die Spule 5A mit der Verarbeitungseinheit 71 B verbindet und dieser die Gleichspannungssignale, d.h. die von der Thermoelementverbindung 21 ermittelten Temperaturmesswerte, zuleitet. Die Verarbeitungseinheit 71 B ist entsprechend zur Verarbeitung der Gleichspannungssignale ausgebildet. Der Pfad 72 A umfasst vorzugsweise zwei Kapazitäten C1, C2, der Pfad 72B umfasst zumindest eine, bevorzugt zwei Spulen L1, L2, jedoch sind selbstverständlich auch andere Ausgestaltungen des Filters 70 möglich.

Die Erfindung ist nicht auf das beschriebene Ausführungsbeispiel beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Medizinisches, insbesondere dentales, Diagnose- oder Behandlungselement (1, 4), umfassend eine Vorrichtung (60, 4A) zum direkten oder indirekten Wirken auf ein Zielgebiet und eine Daten- und / oder Energietransfereinheit (41) zur berührungslosen Daten- und / oder Energieübertragung, wobei die Daten- und / oder Energietransfereinheit (41) zumindest eine erste Spule (5 ― 5C, 6, 6'), Wicklung oder Antenne aufweist, welche zur Daten- und / oder Energieübertragung berührungslos, insbesondere induktiv, mit einer zweiten Spule (5 ― 5C, 6, 6'), Wicklung oder Antenne koppelbar ist, **dadurch gekennzeichnet, dass**
die erste Spule (5 ― 5C, 6, 6'), Wicklung oder Antenne ein erstes Material (M1) aufweist, welches Teil einer Thermoelementverbindung (21) zur Messung eines Temperaturmesswerts des Diagnose- oder Behandlungselements (1, 4) oder eines Bauteils des Diagnose- oder Behandlungselements (1, 4) ist.

2. Medizinisches, insbesondere dentales, Diagnose- oder Behandlungselement (1, 4) nach Anspruch 1, **dadurch gekennzeichnet, dass**
die erste Spule (5 ― 5C, 6, 6'), Wicklung oder Antenne ein zweites Material (M2) aufweist, so dass die Thermoelementverbindung (21) durch die erste Spule (5 ― 5C, 6, 6'), Wicklung oder Antenne gebildet ist.

3. Medizinisches, insbesondere dentales, Diagnose- oder Behandlungselement (1, 4) nach Anspruch 1, **dadurch gekennzeichnet, dass**
ein mit der ersten Spule (5 ― 5C, 6, 6'), Wicklung oder Antenne verbundener elektrischer Leiter (23) ein zweites Material (M2) aufweist, so dass die Thermoelementverbindung (21) durch die erste Spule (5 ― 5C, 6, 6'), Wicklung oder Antenne und den damit verbundenen elektrischen Leiter (23) gebildet ist.

4. Medizinisches, insbesondere dentales, Diagnose- oder Behandlungselement (1, 4) nach einem der vorstehenden Ansprüche, **gekennzeichnet durch** eine Schaltvorrichtung (26) zur wahlweisen Übertragung und / oder Verarbeitung des von der Thermoelementverbindung (21) ermittelten Temperaturmesswerts und der Daten und / oder Energie der berührungslosen Daten- und / oder Energieübertragung.

5. Medizinisches, insbesondere dentales, Diagnose- oder Behandlungselement (1, 4) nach einem der Ansprüche 1 - 3, **gekennzeichnet durch**
eine Vorrichtung (69), welche zur zeitgleichen Ermittlung des Temperaturmesswerts **durch** die Thermoelementverbindung (21) und berührungslosen, insbesondere induktiven, Daten- und / oder Energieübertragung **durch** die erste Spule (5 ― 5C, 6, 6') ausgebildet ist.

6. Medizinisches, insbesondere dentales, Diagnose- oder Behandlungselement (1, 4) nach einem der vorstehenden Ansprüche, **gekennzeichnet durch** zumindest ein gemeinsames Verarbeitungs- und / oder Auswerteelement (23, 27, 28, 46) für den von der Thermoelementverbindung (21) ermittelten Temperaturmesswert und die Daten und / oder Energie der berührungslosen Daten- und / oder Energieübertragung, insbesondere eine gemeinsame Leitung (23), einen gemeinsamen Filter (27), Signalteiler (70), Verstärker (28) oder eine gemeinsame Auswerteschaltung (46).

7. Medizinisches, insbesondere dentales, Diagnose- oder Behandlungselement (1, 4) nach einem der vorstehenden Ansprüche, **dadurch gekennzeichnet, dass** das Diagnose- oder Behandlungselement (1, 4) einen Kopfabschnitt (30) aufweist, von dem die Vorrichtung (60, 4A) zum direkten oder indirekten Wirken auf ein Zielgebiet Materie und / oder Energie auf das Zielgebiet abgibt, wobei die Thermoelementverbindung (21) derart angeordnet ist, dass sie die Temperatur zumindest eines Teils des Kopfabschnitts (30) oder eines Bauteils des Kopfabschnitts (30) misst, wobei die Thermoelementverbindung (21) insbesondere in oder an dem Kopfabschnitt (30) angeordnet ist.

8. Medizinisches, insbesondere dentales, Diagnose- oder Behandlungselement (1, 4) nach einem der vorstehenden Ansprüche, **dadurch gekennzeichnet, dass** das Diagnose- oder Behandlungselement (1, 4) eine Werkzeughalte- und Lösevorrichtung (9) zur lösbaren Verbindung eines Werkzeugs (4) mit dem Diagnose- oder Behandlungselement (1, 4) aufweist, wobei die Thermoelementverbindung (21) derart angeordnet ist, dass sie die Temperatur zumindest eines Teils der Werkzeughalte- und Lösevorrichtung (9) oder im Bereich der Werkzeughalte- und Lösevorrichtung (9) misst.

9. Medizinisches, insbesondere dentales, Diagnose- oder Behandlungselement (1, 4) nach einem der vorstehenden Ansprüche, **gekennzeichnet durch** eine Steuer- und / oder Regelvorrichtung (48), welche ausgebildet ist, den mittels der Thermoelementverbindung (21) ermittelten Temperaturmesswert mit einem Temperaturgrenzwert zu vergleichen und bei Erreichen oder Überschreiten des Temperaturgrenzwerts das Einwirken der Vorrichtung (60, 4A) zum direkten oder indirekten Wirken auf ein Zielgebiet zu unterbrechen, insbesondere einen Antrieb (32) für die Wirkvorrichtung (60, 4A) anzuhalten, und / oder das Erreichen oder Überschreiten des Temperaturgrenzwerts mittels einer Anzeigevorrichtung (49) anzuzeigen.

10. Medizinisches, insbesondere dentales, Diagnose- oder Behandlungselement (1, 4) nach einem der vorstehenden Ansprüche, **gekennzeichnet durch** eine Daten- und / oder Energietransfereinheit (41), welche zur berührungslosen Daten- und / oder Energieübertragung im Hochfrequenz- oder Radiofrequenzbereich ausgebildet ist.

11. Medizinisches, insbesondere dentales, Diagnose- oder Behandlungselement (1, 4) nach einem der vorstehenden Ansprüche, **gekennzeichnet durch** eine Daten- und / oder Energietransfereinheit (41), die ein RFID-Etikett oder einen RFID-Transponder umfasst.

12. Medizinisches, insbesondere dentales, Diagnose- oder Behandlungselement (1, 4) nach einem der vorstehenden Ansprüche, **gekennzeichnet durch** ein mit der Daten- und / oder Energietransfereinheit (41) zur berührungslosen Daten- und / oder Energieübertragung, insbesondere mit der ersten Spule (5 ― 5C, 6, 6'), Wicklung oder Antenne, verbundenes, insbesondere mehrfach beschreibbares, Speicherelement (42, 50), das ausgebildet ist, mittels der Daten- und / oder Energietransfereinheit (41) übermittelbare Daten zu speichern, insbesondere Identifikationsdaten, Betriebsdaten oder Daten über die Reinigung oder Pflege des Diagnose- oder Behandlungselements (1, 4).

13. Medizinische, insbesondere dentale Behandlungsvorrichtung, **gekennzeichnet durch** ein medizinisches, insbesondere dentales, Diagnose- oder Behandlungselement (1, 4) nach einem der vorstehenden Ansprüche und eine zweite Spule (5 ― 5C, 6, 6'), Wicklung oder Antenne, welche zur Daten- und / oder Energieübertragung berührungslos, insbesondere induktiv, mit der ersten Spule (5 ― 5C, 6, 6'), Wicklung oder Antenne koppelbar ist, wobei die zweite Spule (5 ― 5C, 6, 6'), Wicklung oder Antenne bevorzugt an einem mit dem Diagnose- oder Behandlungselement (1, 4) verbindbarem Anschlussteil, Wirkteil, Werkzeug (4) oder Antriebsteil oder an einer von dem Diagnose- oder Behandlungselement (1, 4) separaten Lese- und / oder Sendevorrichtung (52) vorgesehen ist.

14. Verfahren zur Messung eines Temperaturmesswerts mittels eines medizinischen, insbesondere dentalen, Diagnose- oder Behandlungselements (1, 4) gemäß einem der Ansprüche 1 ― 12 oder einer medizinischen, insbesondere dentalen Behandlungsvorrichtung nach Anspruch 13, **dadurch gekennzeichnet, dass** mittels der Thermoelementverbindung (21), die ein erstes Material (M1) der ersten Spule (5 ― 5C, 6, 6'), Wicklung oder Antenne umfasst, ein Temperaturmesswert ermittelt wird und der Temperaturmesswert oder auf Basis des Temperaturmesswerts erhaltene Temperaturdaten mittels der ersten Spule (5 ― 5C, 6, 6'), Wicklung oder Antenne berührungslos, insbesondere induktiv, übertragen werden.

15. Verfahren zur Messung eines Temperaturmesswerts mittels eines medizinischen, insbesondere dentalen, Diagnose- oder Behandlungselements (1, 4) nach Anspruch 14, **dadurch gekennzeichnet, dass**
die Ermittlung des Temperaturmesswerts und die Übertragung des Temperaturmesswerts oder der Temperaturdaten zeitlich versetzt erfolgen.

## Geänderte Patentansprüche

### Geänderte Patentansprüche gemäss Regel 137(2) EPÜ.

**1.** Medizinisches, insbesondere dentales, Diagnose- oder Behandlungselement (1, 4), umfassend eine Vorrichtung (60, 4A) zum direkten oder indirekten Wirken auf ein Zielgebiet und eine Daten- und / oder Energietransfereinheit (41) zur berührungslosen Daten- und / oder Energieübertragung, wobei die Daten- und / oder Energietransfereinheit (41) zumindest eine erste Spule (5 - 5C, 6, 6'), Wicklung oder Antenne aufweist, welche zur Daten- und / oder Energieübertragung berührungslos, insbesondere induktiv, mit einer zweiten Spule (5 - 5C, 6, 6'), Wicklung oder Antenne koppelbar ist, **dadurch gekennzeichnet, dass**
die erste Spule (5 - 5C, 6, 6'), Wicklung oder Antenne ein erstes Material (M1) aufweist, welches Teil einer Thermoelementverbindung (21) des Diagnose- oder Behandlungselements (1, 4) zur Messung eines Temperaturmesswerts des Diagnose- oder Behandlungselements (1, 4) oder eines Bauteils des Diagnose- oder Behandlungselements (1, 4) ist.

**2.** Medizinisches, insbesondere dentales, Diagnose- oder Behandlungselement (1, 4) nach Anspruch 1, **dadurch gekennzeichnet, dass**
die erste Spule (5 - 5C, 6, 6'), Wicklung oder Antenne ein zweites Material (M2) aufweist, so dass die Thermoelementverbindung (21) durch die erste Spule (5 - 5C, 6, 6'), Wicklung oder Antenne gebildet ist.

**3.** Medizinisches, insbesondere dentales, Diagnose- oder Behandlungselement (1, 4) nach Anspruch 1, **dadurch gekennzeichnet, dass**
ein mit der ersten Spule (5 - 5C, 6, 6'), Wicklung oder Antenne verbundener elektrischer Leiter (23) ein zweites Material (M2) aufweist, so dass die Thermoelementverbindung (21) durch die erste Spule (5 - 5C, 6, 6'), Wicklung oder Antenne und den damit verbundenen elektrischen Leiter (23) gebildet ist.

**4.** Medizinisches, insbesondere dentales, Diagnose- oder Behandlungselement (1, 4) nach einem der vorstehenden Ansprüche, **gekennzeichnet durch** eine Schaltvorrichtung (26) zur wahlweisen Übertragung und / oder Verarbeitung des von der Thermoelementverbindung (21) ermittelten Temperaturmesswerts und der Daten und / oder Energie der berührungslosen Daten- und / oder Energieübertragung.

**5.** Medizinisches, insbesondere dentales, Diagnose- oder Behandlungselement (1, 4) nach einem der Ansprüche 1 - 3, **gekennzeichnet durch**
eine Vorrichtung (69), welche zur zeitgleichen Ermittlung des Temperaturmesswerts durch die Thermoelementverbindung (21) und berührungslosen, insbesondere induktiven, Daten- und / oder Energieübertragung **durch** die erste Spule (5 - 5C, 6, 6') ausgebildet ist.

**6.** Medizinisches, insbesondere dentales, Diagnose- oder Behandlungselement (1, 4) nach einem der vorstehenden Ansprüche, **gekennzeichnet durch** zumindest ein gemeinsames Verarbeitungs- und / oder Auswerteelement (23, 27, 28, 46) für den von der Thermoelementverbindung (21) ermittelten Temperaturmesswert und die Daten und / oder Energie der berührungslosen Daten- und / oder Energieübertragung, insbesondere eine gemeinsame Leitung (23), einen gemeinsamen Filter (27), Signalteiler (70), Verstärker (28) oder eine gemeinsame Auswerteschaltung (46).

**7.** Medizinisches, insbesondere dentales, Diagnose- oder Behandlungselement (1, 4) nach einem der vorstehenden Ansprüche, **dadurch gekennzeichnet, dass** das Diagnose- oder Behandlungselement (1, 4) einen Kopfabschnitt (30) aufweist, von dem die Vorrichtung (60, 4A) zum direkten oder indirekten Wirken auf ein Zielgebiet Materie und / oder Energie auf das Zielgebiet abgibt, wobei die Thermoelementverbindung (21) derart angeordnet ist, dass sie die Temperatur zumindest eines Teils des Kopfabschnitts (30) oder eines Bauteils des Kopfabschnitts (30) misst, wobei die Thermoelementverbindung (21) insbesondere in oder an dem Kopfabschnitt (30) angeordnet ist.

**8.** Medizinisches, insbesondere dentales, Diagnose- oder Behandlungselement (1, 4) nach einem der vorstehenden Ansprüche, **dadurch gekennzeichnet, dass** das Diagnose- oder Behandlungselement (1, 4) eine Werkzeughalte- und Lösevorrichtung (9) zur lösbaren Verbindung eines Werkzeugs (4) mit dem Diagnose- oder Behandlungselement (1, 4) aufweist, wobei die Thermoelementverbindung (21) derart angeordnet ist, dass sie die Temperatur zumindest eines Teils der Werkzeughalte- und Lösevorrichtung (9) oder im Bereich der Werkzeughalte- und Lösevorrichtung (9) misst.

**9.** Medizinisches, insbesondere dentales, Diagnose- oder Behandlungselement (1, 4) nach einem der vorstehenden Ansprüche, **gekennzeichnet durch** eine Steuer- und / oder Regelvorrichtung (48), welche ausgebildet ist, den mittels der Thermoelementverbindung (21) ermittelten Temperaturmesswert mit einem Temperaturgrenzwert zu vergleichen und bei Erreichen oder Überschreiten des Temperaturgrenzwerts das Einwirken der Vorrichtung (60, 4A) zum direkten oder indirekten Wirken auf ein Zielgebiet zu unterbrechen, insbesondere einen Antrieb (32) für die Wirkvorrichtung (60, 4A) anzuhalten, und / oder das Erreichen oder Überschreiten des Temperaturgrenzwerts mittels einer Anzeigevorrichtung (49) anzuzeigen.

**10.** Medizinisches, insbesondere dentales, Diagnose- oder Behandlungselement (1, 4) nach einem der vorstehenden Ansprüche, **gekennzeichnet durch** eine Daten- und / oder Energietransfereinheit (41), welche zur berührungslosen Daten- und / oder Energieübertragung im Hochfrequenz- oder Radiofrequenzbereich ausgebildet ist.

**11.** Medizinisches, insbesondere dentales, Diagnose- oder Behandlungselement (1, 4) nach einem der vorstehenden Ansprüche, **gekennzeichnet durch** eine Daten- und / oder Energietransfereinheit (41), die ein RFID-Etikett oder einen RFID-Transponder umfasst.

**12.** Medizinisches, insbesondere dentales, Diagnose- oder Behandlungselement (1, 4) nach einem der vorstehenden Ansprüche, **gekennzeichnet durch** ein mit der Daten- und / oder Energietransfereinheit (41) zur berührungslosen Daten- und / oder Energieübertragung, insbesondere mit der ersten Spule (5 - 5C, 6, 6'), Wicklung oder Antenne, verbundenes, insbesondere mehrfach beschreibbares, Speicherelement (42, 50), das ausgebildet ist, mittels der Daten- und / oder Energietransfereinheit (41) übermittelbare Daten zu speichern, insbesondere ldentifikationsdaten, Betriebsdaten oder Daten über die Reinigung oder Pflege des Diagnose- oder Behandlungselements (1, 4).

**13.** Medizinische, insbesondere dentale Behandlungsvorrichtung, **gekennzeichnet durch** ein medizinisches, insbesondere dentales, Diagnose- oder Behandlungselement (1, 4) nach einem der vorstehenden Ansprüche und eine zweite Spule (5 - 5C, 6, 6'), Wicklung oder Antenne, welche zur Daten- und / oder Energieübertragung berührungslos, insbesondere induktiv, mit der ersten Spule (5 - 5C, 6, 6'), Wicklung oder Antenne koppelbar ist, wobei die zweite Spule (5 - 5C, 6, 6'), Wicklung oder Antenne bevorzugt an einem mit dem Diagnose- oder Behandlungselement (1, 4) verbindbarem Anschlussteil, Wirkteil, Werkzeug (4) oder Antriebsteil oder an einer von dem Diagnose- oder Behandlungselement (1, 4) separaten Lese- und / oder Sendevorrichtung (52) vorgesehen ist.

**14.** Verfahren zur Messung eines Temperaturmesswerts mittels eines medizinischen, insbesondere dentalen, Diagnose- oder Behandlungselements (1, 4) gemäß einem der Ansprüche 1 - 12 oder einer medizinischen, insbesondere dentalen Behandlungsvorrichtung nach Anspruch 13, **dadurch gekennzeichnet, dass** mittels der Thermoelementverbindung (21), die ein erstes Material (M1) der ersten Spule (5 - 5C, 6, 6'), Wicklung oder Antenne umfasst, ein Temperaturmesswert ermittelt wird und der Temperaturmesswert oder auf Basis des Temperaturmesswerts erhaltene Temperaturdaten mittels der ersten Spule (5 - 5C, 6, 6'), Wicklung oder Antenne berührungslos, insbesondere induktiv, übertragen werden.

**15.** Verfahren zur Messung eines Temperaturmesswerts mittels eines medizinischen, insbesondere dentalen, Diagnose- oder Behandlungselements (1, 4) nach Anspruch 14, **dadurch gekennzeichnet, dass**
die Ermittlung des Temperaturmesswerts und die Übertragung des Temperaturmesswerts oder der Temperaturdaten zeitlich versetzt erfolgen.
